# DEMANDE DE BREVET EUROPEEN

(11) **EP 4 397 356 A1**
(43) Date de publication de la demande: **10.07.2024**
(21) Numéro de dépôt: 23209804.6
(22) Date de dépôt: 14.11.2023
(51) Int. Cl.: A61M 39/10, A61G 12/00, F16L 37/12, F16L 37/60, A61M 39/24, A61M 16/08

(54) **PRISE MURALE HOSPITALIÈRE DE DISTRIBUTION DE FLUIDE À CAPOT EMBOITÉ**

(30) Priorité: 03.01.2023 FR 2300018
(71) Demandeur: Air Liquide Medical Systems, 92182 Antony Cedex (FR)
(72) Inventeur: BUSSON, Thibaut, 92182 Antony Cedex (FR); BEAUCHER, Jérôme, 92182 Antony Cedex (FR)
(74) Mandataire: Air Liquide

(57) **Abrégé**

L'invention concerne une prise murale hospitalière de distribution (1) de fluide comprenant un corps de prise (2) d'axe (AA) comprenant une paroi périphérique (4) et une extrémité distale (2-2), et un capot de prise (10) comprenant une ouverture de capot (11) traversée par le corps de prise (2). Le capot de prise (10) comprend plusieurs expansions de paroi (12) se projetant selon l'axe (AA) vers l'extrémité distale (2-2) du corps de prise (2). Les expansions de paroi (12) sont solidaires du corps de capot (2) et comprennent chacune une extrémité libre (12-1) conformée pour coopérer avec la paroi périphérique (4) du corps de prise (2) pour solidariser le capot (10) au corps de prise (2). Gaine (20), bras-plafonnier ou boitier pour prise de distribution de fluide (1) comprenant au moins une prise de distribution (1) de fluide.

## Description

L'invention concerne une prise murale de distribution de fluide avec capot fixé au corps de prise via des expansions de paroi coopérant avec une zone de fixation présente sur la périphérie du corps de prise. La prise peut être directement intégrée à une paroi ou s'insérer dans un boitier, une gaine ou un bras-plafonnier fixé(e) à une paroi ou un plafond comprenant une ou plusieurs prises de distribution de fluide, et être ailleurs utilisée pour distribuer un fluide, tel un gaz médical ou du vide (i.e. dépression ou aspiration).

Les prises murales de distribution de fluide ou plus simplement « prises de fluide » sont couramment utilisées dans les établissements de soins, tels les hôpitaux, les cliniques ou analogues. Elles sont agencées à la sortie des réseaux de fluides, typiquement des canalisations, qui acheminent les fluides médicaux jusque dans les pièces où le ou les fluides sont utilisés, par exemple les salles d'opération et/ou de soins, les chambres de patients ou autres.

Les prises murales servent à distribuer les fluides médicaux qui sont acheminés par les réseaux de fluides aux appareillages ou conduites flexibles venant s'y raccorder, notamment les gaz médicaux, tel l'oxygène, l'air médical ou le protoxyde d'azote (N₂O), le dioxyde de carbone (CO₂) ou encore le vide (i.e. dépression) permettant d'opérer des aspirations notamment de liquides biologiques, par exemple le sang ou d'autres liquides biologiques, pendant les soins des patients.

Les prises murales de distribution de fluide sont aussi couramment appelées 'prises murales', 'prises murales hospitalières' ou analogue car, au sein d'un bâtiment hospitalier, une prise murale de fluide est généralement agencée sur une paroi (mur, cloison...) du bâtiment hospitalier, selon le cas:
- soit directement fixée à ou encastrée dans la paroi,
- soit fixée indirectement à la paroi, en étant agencée dans un boitier ou une gaine qui sont eux-mêmes fixés directement à la paroi du bâtiment hospitalier, par exemple au-dessus de la tête d'un lit d'hôpital,
- soit intégrée dans un module suspendu de type bras-plafonnier hospitalier, lequel peut être fixé à un plafond par exemple.

Autrement dit, une prise murale vient se fixer à une paroi en s'y intégrant directement ou indirectement. Typiquement, une telle prise murale de distribution de fluide comprend un corps de prise traversé par un passage de fluide axial servant à véhiculer le fluide, i.e. du gaz ou du vide. Le passage de fluide relie fluidiquement une extrémité proximale munie d'une tête de prise conformée pour permettre le raccordement à un appareillage ou à une conduite flexible, à une extrémité distale opposée à laquelle vient se raccorder une canalisation de fluide. Un siège de valve et un obturateur de valve mobile, agencés dans le passage principal de fluide, permettent de contrôler les circulations de fluide dans le passage principal, en particulier la fourniture du gaz ou du vide. La tête de prise, qui est située à l'extrémité proximale de la prise murale, fait saillie vers l'extérieur de manière à permettre son raccordement à un appareillage médical utilisant ou véhiculant le fluide, par exemple un appareil ou un dispositif de soins, un tuyau flexible ou analogue.

Des exemples de prises de distribution de fluide sont donnés par FR-A-2628820, EP-A-2306060, EP-A-3932468, EP-A-3922895, EP-A-3922894, EP-A-3922339 et EP-A-3892906.

Lorsqu'elle est non-utilisée, la prise de fluide peut être fermée par un couvercle pivotant ou analogue, servant à protéger la tête de prise contre les chocs, la poussière ou autres.

Une prise comprend également un capot extérieur formant une sorte de bouclier ou paroi servant à protéger la prise contre les chocs et l'encrassement, i.e. dépôts de poussière, projections... pouvant se produire pendant l'utilisation de la prise en milieu hospitalier, par exemple pendant une opération. Ce capot vient se positionner autour de la tête de prise, c'est-à-dire de l'extrémité proximale du corps de prise. Lorsque le capot est mis en position, la tête de prise traverse une ouverture centrale du capot pour faire saillie vers l'extérieur et pouvoir être raccordée à un appareil, un dispositif ou une conduite flexible venant s'y raccorder.

Actuellement, selon le cas, le capot peut être maintenu par un écrou venant se visser sur le corps de prise ou être fixé à la gaine technique ou au boitier au sein duquel est agencée la prise de fluide. Or, ces façons de fixer la prise de fluide posent des problèmes ou engendrent des inconvénients.

Ainsi, lors d'une fixation du capot par écrou, le serrage/vissage de l'écrou est une action qui non seulement est fastidieuse et prend beaucoup de temps sur un chantier de construction mais aussi engendre des risques puisque tout serrage trop fort de l'écrou peut engendre une détérioration du capot et/ou de la façade avant de la gaine au sein de laquelle la prise doit être installée, ou la dissociation de cette façade avant du reste de la gaine, obligeant alors à recommencer les opérations d'installation, ce qui est pénalisant.

Par ailleurs, le positionnement des prises dans une gaine n'est pas forcément parfaitement axé sur les ouvertures de la façade avant de la gaine. Dès lors, lorsque le ou les capots des prises sont fixés directement à la gaine, un tel décalage peut soit engendrer une impossibilité de montage, soit laisser un décentrage apparent entre prise et capot, et donc des défauts de raccordements pouvant engendrer une gêne pour l'utilisateur lors des branchements ou débranchements d'appareillages.

Des systèmes de raccordement fluidiques sont enseignés par US2020/0345997 et US2013/0167841 mais aucun de ceux-ci ne concerne une prise murale hospitalière de distribution de fluide, en particulier de fluides médicaux.

La présente invention vise à résoudre tout ou partie de ces problèmes ou inconvénients, en améliorant et simplifiant la fixation du capot d'une prise murale hospitalière de distribution de fluide, en particulier de fluides médicaux, i.e. gaz ou vide (dépression).

La solution de l'invention concerne alors une prise murale de distribution de fluide, i.e. gaz ou vide, typiquement un prise murale hospitalière, comprenant un corps de prise d'axe (AA) comprenant une paroi périphérique, i.e. externe, un logement interne et une extrémité distale, et un capot de prise comprenant une ouverture de capot traversée par le corps de prise, typiquement une ouverture centrale.

De plus, le corps de prise comprend, agencés dans le logement interne :
- un guide-embout démontable comprenant une pièce-support comprenant un compartiment interne comprenant un clapet de tête coulissant et un moyen élastique agissant sur le clapet de tête en direction d'un premier siège de clapet, et
- une chambre à bille contenant une bille-clapet coopérant avec un second siège de clapet.

En outre, le capot de prise comprend plusieurs expansions de paroi se projetant selon l'axe (AA) vers l'extrémité distale du corps de prise, et lesdites expansions de paroi sont solidaires du corps de capot et comprennent chacune une extrémité libre conformée, i.e. configurée, pour coopérer avec la paroi périphérique du corps de prise pour solidariser le capot au corps de prise.

Selon le mode de réalisation considéré, la prise de distribution de fluide de l'invention peut comprendre l'une ou plusieurs des caractéristiques suivantes :
- la pièce-support est de forme générale tubulaire, typiquement cylindrique.
- la pièce-support est à fond borgne.
- la pièce-support comprend une ou plusieurs membranes de filtration de fluide, c'est-à-dire forme tout ou partie d'une cartouche de filtration.
- les membranes de filtration sont agencées dans la paroi périphérique de la pièce-support.
- le guide-embout démontable, en particulier la pièce-support, comprend en outre une tige-poussoir faisant saillie selon l'axe (AA) en direction de la chambre à bille.
- la tige-poussoir est agencé à l'extérieur du compartiment interne, en particulier sur la surface externe du fond borgne.
- la tige-poussoir coopère avec la bille-clapet de la chambre à bille pour l'éloigner du second siège de clapet et ainsi permettre la circulation de fluide.
- la tige-poussoir vient appuyer sur la bille-clapet pour l'éloigner du second siège de clapet, lorsque le guide-embout est agencé dans le logement interne du corps de prise.
- au moins une partie du logement interne du corps de prise forme un passage de fluide interne.
- le logement interne du corps de prise est aménagé axialement selon l'axe (AA) dans le corps de prise.
- la pièce-support comprend une partie avant comprenant l'orifice de sortie de gaz.
- le corps de prise comprend une extrémité proximale munie d'une tête de prise dirigée vers l'extérieur.
- le corps de prise a une forme allongée s'étend selon l'axe (AA).
- l'extrémité proximale est située à l'avant du corps de prise, c'est-à-dire du côté fournissant ou distribuant le fluide, i.e. gaz ou vide.
- l'extrémité distale est située à l'arrière du corps de prise, c'est-à-dire du côté amenant le fluide, i.e. gaz ou vide.
- le passage de fluide interne s'étend axialement entre l'extrémité distale et l'extrémité proximale,
- le passage de fluide interne débouche au niveau de la tête de prise.
- la tête de prise comprend un orifice de fluide, i.e. de fourniture de gaz ou permettant l'aspiration par le vide.
- le capot de prise comprend en outre une face avant dirigée vers l'extérieur et une face arrière, opposée à la face avant, dirigée vers l'extrémité distale dudit corps de prise.
- le capot de prise a une forme de disque.
- le capot de prise a une forme de disque plat ou bombé.
- le capot de prise comprend un plan P perpendiculaire à l'axe (AA).
- les expansions de paroi comprennent chacune une extrémité libre portant au moins un épaulement radial dirigé vers l'axe (AA).
- les expansions de paroi forment des pattes ou languettes d'accrochage, i.e. de fixation, d'arrimage, de maintien ou analogue.
- le capot de prise comprend au moins 3 expansions de paroi réparties autour de l'ouverture de capot, de préférence de 4 à 8 expansions de paroi, par exemple 4 expansions de paroi.
- les expansions de paroi sont séparées les unes des autres par des parois intercalaires, c'est-à-dire que le capot de prise comprend une alternance d'expansions de paroi et de parois intercalaires.
- les expansions de paroi sont angulairement réparties autour de l'ouverture de capot, i.e. l'orifice central.
- le capot de prise comprend 4 expansions de paroi situées à 90° les unes des autres.
- le capot de prise comprend 4 expansions de paroi séparées par 4 parois intercalaires.
- les expansions de paroi sont flexibles, c'est-à-dire qu'elles présentent une élasticité suffisante pour permettre leur insertion et leur arrimage autour du corps de prise.
- les parois intercalaires sont rigides.
- les expansions de paroi peuvent être identiques les unes aux autres, c'est-à-dire avoir des formes et dimensions identiques, ou peuvent être différentes, c'est-à-dire que les expansions de paroi peuvent avoir plusieurs formes et/ou plusieurs dimensions.
- les expansions de paroi ont une longueur de 5 à 15 mm, de préférence de l'ordre de 8 à 12 mm.
- l'épaulement radial de chaque expansion de paroi a une taille de 1 à 4 mm, c'est-à-dire la partie formant crochet ou analogue.
- chaque expansion de paroi a une largeur comprise entre 3 et 10 mm.
- les expansions de paroi sont angulairement espacées les unes des autres.
- les expansions de paroi sont portées par une embase annulaire agencée autour de l'ouverture de capot, sur la face arrière du capot, c'est-à-dire une structure renforcée agencée coaxialement à l'ouverture de capot.
- les parois intercalaires sont aussi portées par l'embase annulaire agencée autour de l'ouverture de capot.
- ledit au moins un épaulement radial vient coopérer avec au moins une structure de fixation portée par la paroi périphérique du corps de prise pour solidariser le capot au corps de prise.
- les parois intercalaires ne portent pas d'épaulement radial, donc ne viennent pas coopérer avec la structure de fixation portée par la paroi périphérique du corps de prise et ne participent pas à solidarisation du capot au corps de prise.
- la structure de fixation portée par la paroi périphérique du corps de prise comprend au moins une gorge (ou rainure) annulaire ou au moins une paroi annulaire, c'est-à-dire un élément en relief, aménagée sur toute ou partie du pourtour du corps de prise.
- au moins un épaulement radial d'au moins une expansion de paroi du capot de prise vient en butée contre au moins une structure de fixation portée par la paroi périphérique du corps de prise pour empêcher tout retrait du capot de prise du corps de prise.
- la structure de fixation portée par la paroi périphérique du corps de prise comprend un filetage, c'est-à-dire une pluralité de gorges ou rainures annulaires sensiblement parallèles formant le filet dudit filetage.
- le corps de capot et les expansions, et les parois intercalaires éventuelles, de paroi sont formés d'une seule pièce, de préférence par moulage-injection d'un matériau polymère.
- le corps de prise est en métal, par exemple en alliage de cuivre ou en acier inoxydable.
- le corps de prise comprend un guide-embout démontable.
- le guide-embout comprend une pièce-support de forme générale tubulaire à fond borgne, c'est-à-dire fermée à une extrémité, comprenant une ou plusieurs membranes de filtration de fluide, et un orifice au travers duquel le fluide (i.e. gaz ou vide) peut circuler, c'est-à-dire entrer ou sortir de l'élément de filtration.
- les membranes de filtration du guide-embout comprennent des pores (aussi appelée mailles) ayant une taille comprise entre 50 et 300 µm, de préférence entre 50 et 80 µm, en particulier lorsqu'elles sont destinées à filtrer un gaz, notamment un gaz médical.
- le clapet de tête coopère avec le premier siège de clapet pour contrôler la circulation du fluide dans le guide-embout.
- le moyen élastique du guide-embout comprend ou est un ressort.
- le moyen élastique repousse le clapet de tête en direction du premier siège de clapet.
- la chambre à bille est extractible.
- la chambre à bille est visée dans le corps de prise, en particulier dans le passage central du corps de prise.
- elle comporte des moyens de fixation à une cloison, un mur ou analogue.
- alternativement, elle comporte des moyens de fixation, i.e. d'intégration, à/dans une gaine, un bras-plafonnier ou un boitier de prise de distribution de fluide.

L'invention porte aussi sur une gaine, un bras-plafonnier ou un boitier de prise de distribution de fluide comprenant au moins une prise de distribution de fluide selon l'invention. La ou les prises de distribution de fluide sont reliées à une canalisation de gaz ou de vide, en particulier au réseau de canalisations de gaz ou de vide (i.e. dépression) d'un bâtiment hospitalier.

Autrement dit, la gaine, le bras-plafonnier ou le boitier sont agencés dans un établissement hospitalier, en particulier sur un mur, une cloison ou analogue.

Par ailleurs, le fluide est de l'oxygène, de l'air ou du protoxyde d'azote.

Par ailleurs, l'invention concerne aussi une utilisation d'une prise de distribution de fluide selon l'invention pour fournir un fluide à un appareil ou à un dispositif médical, en particulier un gaz ou du vide (i.e. dépression ou aspiration).

Lors d'une opération d'installation, montage ou maintenance d'une prise de fluide, la mise en place du capot de prise sur la prise, se fait simplement par insertion de l'extrémité proximale de la prise qui porte la tête de prise, dans l'ouverture centrale du corps de capot.

Les expansions de paroi situées sur la face arrière du capot, translatent alors en direction de la structure de fixation portée par la paroi périphérique du corps de prise, i.e. gorge, rainure ou paroi annulaire, jusqu'à venir coopérer avec cette structure de fixation via leur épaulement radial, en particulier l'épaulement radial de chaque expansion de paroi vient en butée contre une structure de fixation réciproque portée par le corps de prise de manière à assurer une fixation du capot au corps de prise, c'est-à-dire via une sorte de « clipsage » ou emboitement, et s'opposer à tout retrait ou détachement ultérieur non souhaité de ces éléments.

L'invention va maintenant être mieux comprise grâce à la description détaillée suivante, faite à titre illustratif mais non limitatif, en référence aux figures annexées parmi lesquelles :
Fig. 1 représente une gaine de distribution de fluides comprenant plusieurs prises de distribution de fluide,
Fig. 2 représente un mode de réalisation d'un capot destiné à équiper une prise de distribution de fluide selon l'invention,
Fig. 3 montre la partie distale d'une prise de distribution de fluide selon l'invention équipée du capot de prise de Fig. 2,
Fig. 4 montre la partie proximale d'une prise de distribution de fluide selon l'invention équipée du capot de prise de Fig. 2,
Fig. 5 monte une prise de distribution de fluide selon l'invention équipée du capot de prise de Fig. 2 agencée dans une gaine pour prises de distribution de fluide, comme celle de Fig. 1,
Fig. 6 est une vue de la face arrière du capot de prise de Fig. 2,
Fig. 7 est une vue de ¾ de la face arrière du capot de prise de Fig. 2, et
Fig. 8 schématise la solidarisation du capot de prise sur le corps de prise d'une prise de distribution de fluide selon l'invention.
Fig. 9 schématise un mode de réalisation d'une prise de distribution de fluide (vue en coupe) autour de laquelle peut venir se solidariser un capot de prise selon l'invention.

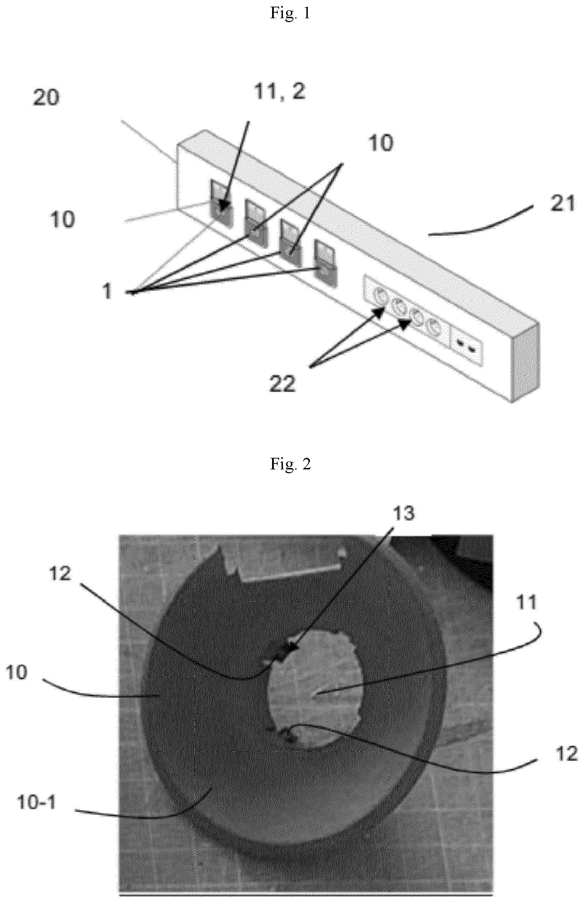

Fig. 1 représente une gaine 20 de distribution de fluides comprenant ici 4 prises de distribution de fluide 1 juxtaposées permettant de fournir des gaz médicaux et du vide, c'est-à-dire une dépression (< 1 atm), au sein d'un bâtiment hospitalier, par exemple dans une chambre de soins ou une salle d'opérations.

Les prises de fluide 1 sont encastrées dans la gaine 20 qui a une forme de boitier allongé, et qui est elle-même fixée à un mur 21 ou une paroi du bâtiment hospitalier. La gaine 20 peut aussi comprendre d'autres éléments, comme des prises de raccordement électrique 22.

Avantageusement, les 4 prises fournissent de l'air médical, de l'oxygène médical, du protoxyde d'azote ou du MEOPA, i.e. mélange équimolaire (50% mol./50% mol.) d'oxygène et de protoxyde d'azote (N₂O), et du vide. Bien entendu, elle peut être utilisée pour fournir un autre gaz ou mélange gazeux.

Chaque prise 1 est munie d'un capot de prise 10 comprenant une ouverture centrale 11 traversée par le corps de prise 2, en particulier le capot de prise 10 selon l'invention tel que décrit ci-dessous. Ce capot 10 extérieur forme une sorte de bouclier avant servant à protéger la prise 1 contre les chocs et l'encrassement, i.e. dépôts de poussière, projections... pouvant se produire pendant l'utilisation de la prise 1 considérée, par exemple pendant les soins d'un patient.

La gaine 20 peut recevoir des prises de fluides 1 selon l'invention, telle celle présentée en Fig. 2 à Fig. 8 et décrite ci-après.

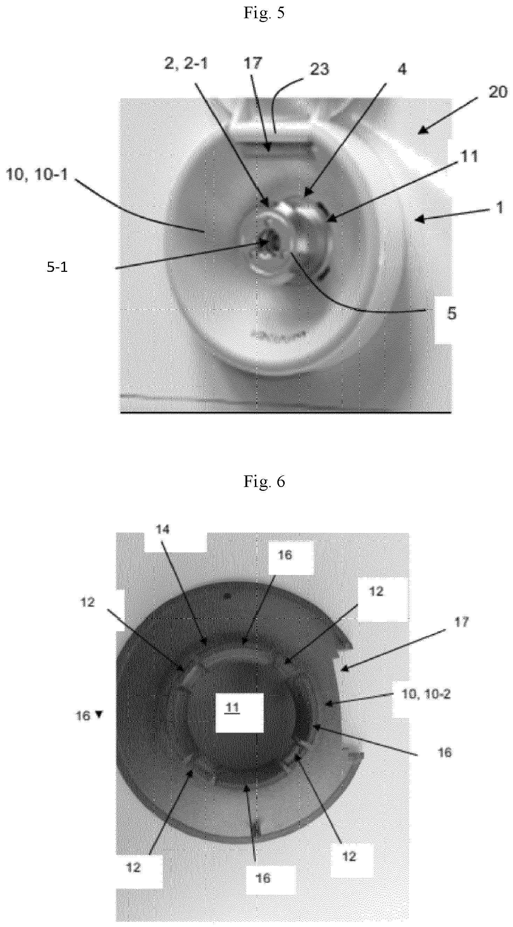

Fig. 5 est une vue grossie d'une des prises 1 de fluide agencées dans la gaine 20 de Fig. 1. La prise 1 est équipée d'un capot de prise 10 selon l'invention, dont l'architecture est détaillée ci-après en relation avec Fig. 2 à Fig. 4, Fig. 6 et Fig. 7 qui proposent un mode de réalisation d'un capot 10 pour prise de fluide 1 selon l'invention.

Ainsi, Fig. 2, Fig. 6 et Fig. 7 montrent le capot 10 détaché de la prise 1, c'est-à-dire avant sa fixation ou sa solidarisation à la prise de fluide 1.

Le capot de prise 10 comprenant une ouverture de capot 11, c'est-à-dire une ouverture centrale, et a ici une forme générale de disque plat ou bombé. Il comprend deux faces opposées 10-1, 10-2, à savoir une face avant 10-1, ou façade, dirigée vers l'extérieur et une face arrière 10-2, ou dos, opposée à la face avant 10-1, c'est-à-dire arrière vers l'arrière de la prise 1, i.e. vers le mur 21.

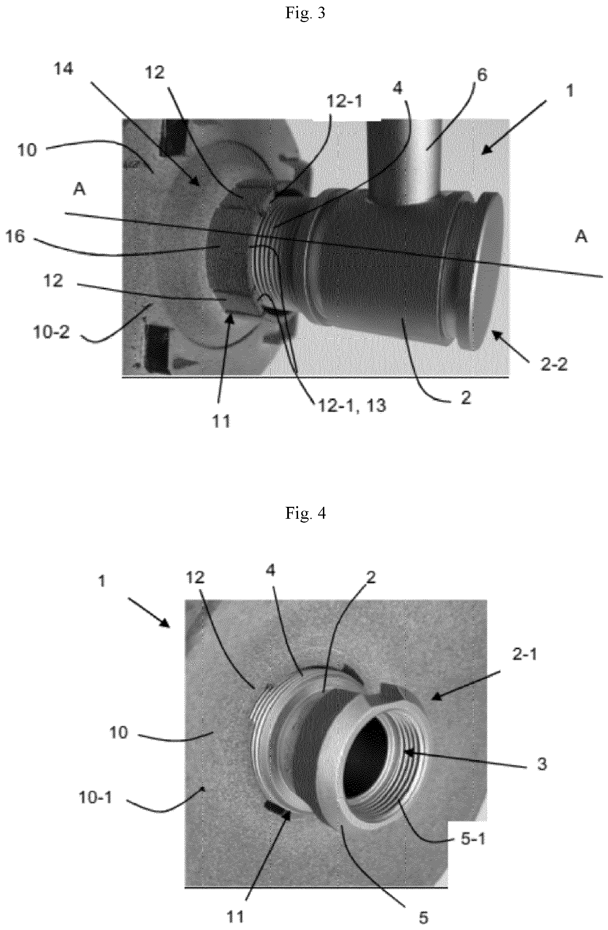

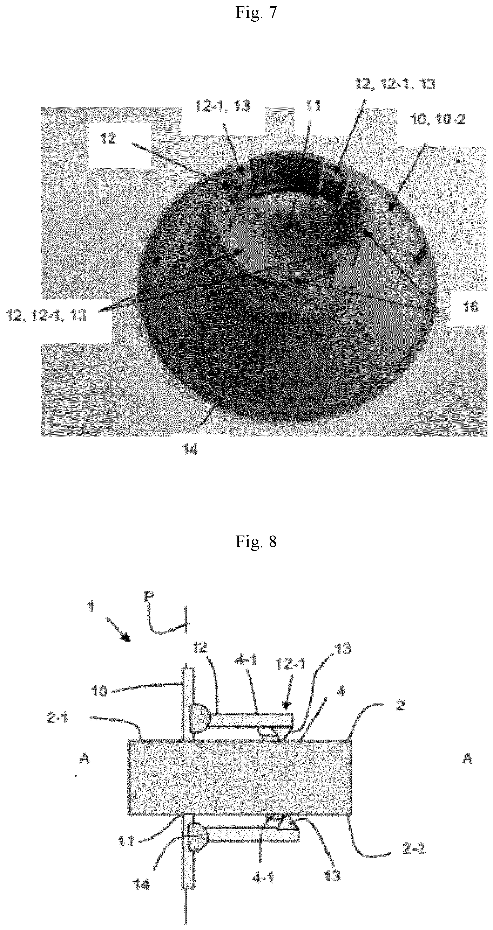

Le capot de prise 10 comprend plusieurs expansions de paroi 12 qui sont solidaires du corps de capot 2, par exemple au moins 4 expansions de paroi 12, comme mieux visible sur Fig. 6 et Fig. 7. Elles comprennent chacune une extrémité libre 12-1 conformée ou configurer pour coopérer avec la paroi périphérique 4 du corps de prise 2 pour solidariser le capot 10 au corps de prise 2, comme illustré sur Fig. 3 et Fig. 4.

Ici, le capot de prise 10 comprend 4 expansions de paroi 12 angulairement réparties autour de l'ouverture centrale 11 (et de l'axe XX), c'est-à-dire décalées angulairement les unes des autres d'environ 90°. Dans le mode de réalisation présenté, les 4 expansions de paroi 12 sont séparées les unes des autres par des parois intercalaires 16 de forme arquée, comme visible sur Fig. 6 et Fig. 7, à savoir ici 4 parois intercalaires 16.

Dans d'autres modes de réalisation (non schématisés), il pourrait y avoir davantage de parois intercalaires 16 entre les expansions de paroi 12, voire même aucune parois intercalaires 16 et donc uniquement des expansions de paroi 12.

On voit sur Fig. 3 et Fig. 7 que les expansions de paroi 12 se projettent à l'arrière du corps 2 de capot de prise 10 et s'étendent selon l'axe (AA), en direction de l'extrémité distale 2-2 du corps de prise 2, lorsque le capot 2 a été enfilé ou inséré à travers de l'ouverture centrale 11 du capot 2, c'est lorsque le corps de prise 2 traverse l'ouverture centrale 11.

Les expansions de paroi 12 forment des pattes ou languettes d'accrochage, de fixation, d'arrimage, de maintien ou analogue. Pour ce faire, elles comprennent chacune une extrémité libre 12-1 portant (au moins) un épaulement radial 13 dirigé vers l'axe (AA), c'est-à-dire une petite structure en relief se projetant vers le corps de prise 2, comme schématisé en Fig. 8. L'épaulement 13 est aménagé sur toute ou partie de la largeur de l'extrémité 12-1 des expansions de paroi 12.

Les expansions de paroi 12, telles des pattes ou languettes, ont une structure en forme de bandelette allongée qui peut être plane ou légèrement incurvée pour épouser le contour externe du corps de prise 2 de forme cylindrique.

Les épaulements radiaux 13 constituent des 'crochets' ou `griffes' d'arrimage ou similaire. Chaque épaulement radial 13 vient coopérer avec la paroi périphérique 4 du corps de prise 2, c'est-à-dire sa paroi externe, pour solidariser le capot 10 au corps de prise 2 et empêcher ou s'opposer à son retrait du corps de prise 2. Ici, le corps de prise 2 a une forme générale tubulaire, comme visible en Fig. 3.

Préférentiellement, les expansions de paroi 12, formant des pattes ou languettes d'arrimage, sont légèrement flexibles pour faciliter leur mise en place par emboitement ou analogue autour du corps de prise 2. Leur flexibilité ou élasticité relative est conférée par la forme en bandelette et le matériau polymère dont les expansions de paroi 12 sont formées.

A l'inverse, les parois intercalaires 16 situées entre les expansions de paroi 12 ne comportent pas d'épaulements radiaux 13 constituant des 'crochets' ou 'griffes' d'arrimage ou similaire. Préférentiellement, les parois intercalaires 16 sont plus rigides que les expansions de paroi 12 flexibles.

A titre indicatif, les expansions de paroi peuvent avoir une longueur de 5 à 15 mm, de préférence de l'ordre de 8 à 12 mm, l'épaulement radial de chaque expansion de paroi une taille de 1 à 4 mm, et chaque expansion de paroi une largeur comprise entre 3 et 10 mm.

On voit aussi en Fig. 8 que le capot de prise 10 comprend un plan P, par exemple un plan P passant par le corps de capot lorsqu'il est plat, qui est perpendiculaire à l'axe (AA) du corps de prise 2.

Pour améliorer l'accrochage, on aménage une structure de fixation 4-1, de préférence une (ou des) gorge annulaire ou une (des) paroi annulaire, dans ou sur la paroi périphérique 4, sur toute ou partie du pourtour du corps de prise 2 (i.e. autour du corps cylindrique), par exemple une (ou des) gorge ou rainure annulaire, de sorte que l'épaulement 13 des expansions de paroi 12 du capot de prise 10 vienne en butée contre cette structure de fixation 4-1 portée par la paroi périphérique 4 du corps de prise 2 pour empêcher tout retrait du capot de prise 10 du corps de prise 2.

De préférence, les expansions de paroi 12, les parois intercalaires 16 optionnelles, et le reste du capot 10 sont formées d'une seule pièce, de préférence par moulage-injection d'un matériau polymère, alors que le corps de prise 2 est quant à lui usiné en métal, par exemple en alliage de cuivre ou en acier inoxydable.

Fig. 3 montre la partie distale 2-2 de la prise de distribution de fluide 1, c'est-à-dire sa partie arrière, équipée du capot de prise 10 selon l'invention.

On voit qu'ici, les épaulements 13 des expansions de paroi 12 viennent coopérer, i.e. en butée, contre un filetage ou analogue constituant la structure de fixation 4-1, lequel est aménagé autour de la paroi périphérique 4 du corps de prise 2. Ce filetage comprend un filet, c'est-à-dire schématiquement une succession de gorges ou rainures agencées sensiblement parallèles les unes ou autres.

Les parois intercalaires 16 optionnelles ne coopèrent pas, quant à elles, avec la structure de fixation 4-1 aménagée autour de la paroi périphérique 4 du corps de prise 2.

Autrement dit, les épaulements 13 des expansions de paroi 12 viennent s'accrocher au filet du filetage formant la structure de fixation 4-1 du corps de prise 2 mais pas les parois intercalaires 16 optionnelles.

On voit sur Fig. 6 et Fig. 5, que le capot de prise 10 comprend un rebord périphérique qui n'est pas totalement circulaire puisqu'il comporte une découpe ou échancrure 17 permettant de recevoir la charnière 23 du couvercle de prise pivotant (non montré), comme illustré en Fig. 5.

On note, par ailleurs, que, dans le mode de réalisation proposé, les expansions de paroi 12, c'est-à-dire languettes ou analogue, et les parois intercalaires 16 sont portées, c'est-à-dire fixé à leur extrémité non-libre, par une embase annulaire 14 agencée autour de l'ouverture 11 de capot, sur la face arrière du capot 10-2, c'est-à-dire une structure renforcée situé à la base des expansions de paroi 12 et agencée coaxialement à l'ouverture centrale 11 du capot 10.

En d'autres termes, selon l'invention, le capot 2 peut schématiquement venir se « clipser », c'est-à-dire s'emboiter, directement sur le corps 2 de la prise 1 de fluide et les épaulements radiaux 13 formant des crochets ou griffes d'arrimage ou similaires, viennent s'arrimer ou s'accrocher sur le filetage extérieur 4-1 de la prise 1.

Lorsqu'on souhaite retirer le capot 10, il convient de le « déclipser », i.e. désemboiter, au moyen d'un outil adapté venant écarter les extrémités des expansions de paroi 12 de la paroi 4 du corps de prise 2 de manière à éloigner les épaulements radiaux 13 formant des crochets ou griffes d'arrimage de la structure de fixation 4-1 et les dissocier de ladite structure de fixation 4-1 du corps de prise 2, à savoir ici le filetage 4-1.

Par ailleurs, une tubulure 15 permet de raccorder fluidiquement le corps de prise 2 à une canalisation d'amenée de fluide, par exemple au réseau hospitalier, afin d'alimenter la prise 1 en fluide, i.e. gaz ou vide. Un tel raccordement est classique et non-détaillé ici.

Fig. 4 montre la partie proximale 2-1 de la prise de fluide 1, i.e. gaz ou vide, équipée du capot 10 selon l'invention. On y voit la face avant 10-1 du capot 1 qui est dirigée vers l'extérieur, c'est-à-dire vers la tête de prise 5 faisant saille vers l'extérieur, à laquelle vient se raccorder un appareillage médical (non montré).

La tête de prise 5 comprend l'orifice de sortie 5-1 de la prise 1 par lequel sort le gaz ou permettant l'aspiration par le vide (i.e. dépression). L'orifice de sortie 5-1 de la prise est situé à l'extrémité proximale

Plus généralement, le corps de prise 2 a une architecture interne et un fonctionnement similaires ou identiques à ceux décrits par EP-A-3892906.

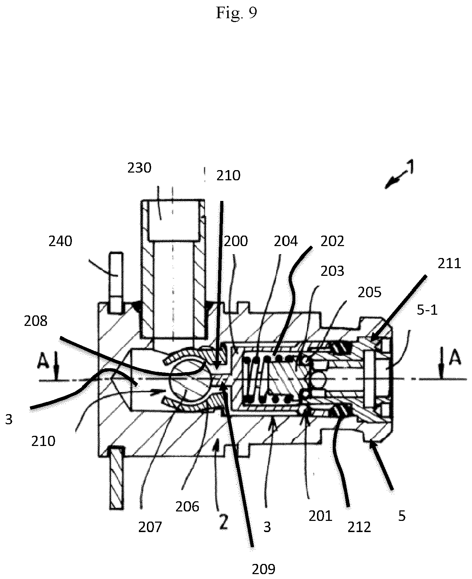

Plus précisément, comme illustré sur Fig. 9, le corps de prise 2 comprend un guide-embout 200 démontable comprenant une pièce-support 201 creuse de forme générale tubulaire (i.e. cylindrique) à fond borgne, c'est-à-dire comprenant un compartiment interne fermé à une extrémité, comprenant des membranes de filtration de fluide, et un passage interne 202 au travers duquel le fluide, i.e. du gaz, peut circuler. Le guide-embout 200 comprenant l'orifice de sortie de gaz 5-1.

Les membranes de filtration agencées dans la paroi périphérique de la pièce-support 201 de forme générale tubulaire ont des pores ou mailles de tailles comprises entre 50 et 300 µm, de préférence entre 50 et 80 µm. Elles sont traversées par le fluide qui les traverse lors de son cheminement par le guide-embout 200.

Selon un mode de réalisation, la pièce-support 201 du guide-embout 200 comprend une partie avant 211 comprenant l'orifice de sortie de gaz 5-1. Cette partie avant 211 peut être démontable du reste de la pièce-support 201 du guide-embout 200.

Le guide-embout 200 comprend en outre, agencés dans son logement ou compartiment interne 202, un clapet de tête coulissant 203 et un moyen élastique 204, tel un ressort cylindrique ou analogue, le moyen élastique 204 agissant sur, i.e. repoussant, le clapet de tête 203 en direction d'un premier siège de clapet 205 permettant de contrôler la circulation du fluide au sein du guide-embout 200.

Le clapet de tête 203 coulisse selon l'axe AA dans le logement interne 7, i.e. coulissant en translation, au sein dudit compartiment interne de la pièce-support 201 creuse de forme générale tubulaire.

Par ailleurs, le corps de prise 2 comprend aussi une chambre à bille 206 contenant une bille-clapet 207 coopérant avec un second siège de clapet 208 et une tige-poussoir 209 portée par la pièce-support 201. La tige-poussoir 209 est portée par la surface extérieure arrière de la pièce-support 201.

La chambre à bille 206, de préférence extractible, est visée dans le fond du corps de prise 2, en particulier dans la partie amont du logement interne 3 formant un passage axial du corps de prise 2. La chambre à bille 206 est donc prise en sandwich entre le fond de prise et le guide-embout 200 extractible.

La chambre à bille 206 est ouverte à son extrémité arrière 210 et en communication fluidique avec un conduit 230 amenant le fluide, lequel est raccordé fluidiquement au réseau de canalisations de fluide d'un bâtiment hospitalier.

La bille-clapet 207 et le second siège de clapet 208 permettent de contrôler la circulation de fluide dans le corps de prise 2, par exemple de gaz, provenant du conduit 230 et circulant vers le guide-embout 200 afin d'assurer la fourniture de fluide à un appareillage (non montré) raccordé à la prise 1 de fluide, par exemple d'oxygène ou d'air médical.

Le corps de prise 2 a une forme allongée selon un axe longitudinal AA, lequel est traversé axialement par le passage interne formant un logement interne 3 au sein duquel sont agencés le guide-embout 200 et la chambre à bille 206. Afin d'assurer une fixation du guide-embout 200 au sein du passage ou logement 3 du corps 2 de la prise de distribution de fluide 1, celui-ci comprend un filetage périphérique externe venant coopérer avec un taraudage aménagé dans la paroi du passage 3 du corps de prise 2.

L'étanchéité fluidique entre le guide-embout 200 et le corps de prise 2 est assurée par des joints toriques 212 ou analogues.

En fonctionnement, le guide-embout 200 vient appuyer sur la bille-clapet 207, par l'intermédiaire de la tige-poussoir 209 portée par la pièce-support 201, pour la décoller du siège de clapet 208 et autoriser ainsi une communication fluidique entre le conduit 230, le fond du passage axial interne 3 du corps de prise 2 et les passages internes de la chambre à bille 206 et du guide-embout 200 afin d'assurer une circulation du gaz du conduit 230 en direction de l'orifice de sortie 5-1 de la prise 1. La tige-poussoir 11 est une expansion axiale faisant saillie sur la surface externe arrière du guide-embout 200, i.e. de la pièce-support 201, et solidaire de celui-ci. Elle traverse axialement un canal de liaison 210 agencé dans la paroi de la chambre à bille 206 et vient appuyer sur la bille-clapet 206 pour la décoller de son siège.

Selon un mode de réalisation, la prise de fluide 1 de l'invention peut être insérée dans un boitier de prise pouvant recevoir une seule prise 1 à la fois, par exemple un boitier de prise comme décrit par EP-A-3932468, ou encore une gaine 20 ou un bras-plafonnier, ou être directement fixée à une paroi 240, tel un mur, une cloison ou analogue, d'un établissement hospitalier, par l'intermédiaire de moyens de fixation à une cloison ou analogue (non détaillés).

D'une façon générale, une prise de distribution 1 de fluide munie d'un capotage 10 « clipsable » selon l'invention, présente notamment les avantages suivants :
- une fixation très rapide du capot 2 sur le corps 2 de la prise 1,
- aucun risque de déformer ou « déclipser » la gaine 20 ou de détériorer le capot 2, lors du montage.

Une prise de distribution 1 de fluide selon l'invention est particulièrement bien adaptée à une utilisation pour fournir un fluide, tel du gaz ou du vide, à un appareil ou à un dispositif médical au sein d'un bâtiment hospitalier, par exemple dans une salle de soins, d'opérations, d'éveil ou une chambre de patient.

## Revendications

1. Prise murale de distribution (1) de fluide comprenant :
- un corps de prise (2) d'axe (AA) comprenant une paroi périphérique (4), un logement interne (3) et une extrémité distale (2-2), et
- un capot de prise (10) comprenant une ouverture de capot (11) traversée par le corps de prise (2),
et dans laquelle le corps de prise (2) comprend, agencés dans le logement interne (3):
- un guide-embout démontable (200) comprenant une pièce-support (201) comprenant un compartiment interne (202) comprenant un clapet de tête (203) coulissant et un moyen élastique (204) agissant sur le clapet de tête (203) en direction d'un premier siège de clapet (205), et
- une chambre à bille (206) contenant une bille-clapet (207) coopérant avec un second siège de clapet (208),
**caractérisée en ce que** :
- le capot de prise (10) comprend plusieurs expansions de paroi (12) se projetant selon l'axe (AA) vers l'extrémité distale (2-2) du corps de prise (2), et
- les expansions de paroi (12) sont solidaires du corps de capot (2) et comprennent chacune une extrémité libre (12-1) conformée pour coopérer avec la paroi périphérique (4) du corps de prise (2) pour solidariser le capot (10) au corps de prise (2).

2. Prise selon la revendication 1, **caractérisée en ce que** le corps de prise (2) d'axe (AA) comprend une extrémité proximale (2-1) munie d'une tête de prise (5) dirigée vers l'extérieur.

3. Prise selon la revendication 1, **caractérisée en ce que** les expansions de paroi (12) sont angulairement réparties et séparées les unes des autres par des parois intercalaires (16).

4. Prise selon la revendication 1, **caractérisée en ce que** le capot de prise (10) comprend en outre une face avant (10-1) dirigée vers l'extérieur et une face arrière (10-2), opposée à la face avant (10-1), dirigée vers l'extrémité distale (2-2) dudit corps de prise (2).

5. Prise selon la revendication 1, **caractérisée en ce que** les expansions de paroi (12) comprennent une extrémité libre (12-1) portant au moins un épaulement radial (13) dirigé vers l'axe (AA), ledit au moins un épaulement radial (13) venant coopérer avec au moins une structure de fixation (4-1) portée par la paroi périphérique (4) du corps de prise (2) pour solidariser le capot (10) au corps de prise (2).

6. Prise selon la revendication 5, **caractérisée en ce que** la structure de fixation (4-1) portée par la paroi périphérique (4) du corps de prise (2) comprend au moins une gorge annulaire ou au moins une paroi annulaire aménagée sur toute ou partie du pourtour du corps de prise (2).

7. Prise selon l'une des revendications 5 ou 6, **caractérisée en ce qu'**au moins un épaulement radial (13) d'au moins une expansion de paroi (12) du capot de prise (10) vient en butée contre au moins une structure de fixation (4-1) portée par la paroi périphérique (4) du corps de prise (2) pour empêcher tout retrait du capot de prise (10) du corps de prise (2).

8. Prise selon la revendication 1, **caractérisée en ce que** le corps de capot (2) et les expansions de paroi (12) sont formés d'une seule pièce, de préférence par moulage-injection d'un matériau polymère.

9. Prise selon la revendication 1, **caractérisée en ce que** le guide-embout (200) comprend un orifice de sortie de gaz (5-1).

10. Prise selon la revendication 1, **caractérisée en ce que** le guide-embout (200) comprend en outre une tige-poussoir (209) faisant saillie selon l'axe (AA) en direction de la chambre à bille (206).

11. Prise selon la revendication 10, **caractérisée en ce que** la tige-poussoir (209) coopère avec la bille-clapet (207) de la chambre à bille (206) pour l'éloigner du second siège de clapet (208).

12. Gaine (20), bras-plafonnier ou boitier pour prise de distribution de fluide (1 ) comprenant au moins une prise murale de distribution (1) de fluide selon l'une des revendications précédentes.

13. Utilisation d'une prise de distribution (1) de fluide selon l'une des revendications 1 à 11 ou d'une gaine (20), d'un bras-plafonnier ou d'un boitier selon la revendication 12 pour fournir un fluide à un appareil ou à un dispositif médical, en particulier un gaz ou du vide.

14. Utilisation selon la revendication 13, **caractérisée en ce que** la gaine (20), le bras-plafonnier ou le boitier sont agencés dans un établissement hospitalier, en particulier sur un mur, une cloison ou analogue.

15. Utilisation selon la revendication 13, **caractérisée en ce que** le fluide est de l'oxygène, de l'air ou du protoxyde d'azote.
